# EUROPÄISCHE PATENTSCHRIFT

(11) **EP 1 153 578 B1**
(45) Veröffentlichungstag und Bekanntmachung des Hinweises auf die Patenterteilung: **21.03.2007**
(21) Anmeldenummer: 01111616.7
(22) Anmeldetag: 12.05.2001
(51) Int. Cl.: A61B 18/14

(54) **Scheren- oder zangenförmiges chirurgisches Instrument**
Forceps- or scissor-like surgical instrument
Instrument chirurgical en forme de pince ou de ciseaux

(30) Priorität: 13.05.2000 DE 10023534
(43) Veröffentlichungstag der Anmeldung: 14.11.2001
(73) Patentinhaber: Aesculap AG & Co. KG, 78532 Tuttlingen (DE)
(72) Erfinder: Mayenberger, Rupert, Dipl.-Ing., 78239 Rielasingen (DE); Rothweiler, Christoph, Dipl.-Ing., 78166 Donaueschingen (DE); Weisshaupt, Dieter, Dipl.-Ing., 78194 Immendingen (DE)
(74) Vertreter: Boehme, Ulrich

(56) Entgegenhaltungen:
- EP-A- 0 931 515
- DE-A- 19 828 976
- DE-C- 19 729 461
- US-A- 5 976 132

## Beschreibung

Die Erfindung betrifft ein scheren- oder zangenförmiges chirurgisches Instrument gemäß dem Oberbegriff des Anspruchs 1. Ein solches Instrument ist z.B aus US.5 976 132 bekannt.

Ein anderes Instrument ist beispielsweise aus der DE 198 28 976 A bekannt. Die beiden Branchen fungieren dabei gleichzeitig als elektrische Zuleitungen, mit jeder Branche ist ein elektrisches Kabel verbunden, die beiden Branchen sind im Bereich des Schlusses, also der Schwenklagerung, durch eine speziell ausgebildete Isolierhülse elektrisch voneinander isoliert.

Bei einer solchen Ausgestaltung ist problematisch, daß beide Branchen jeweils mit einem Zuleitungskabel versehen sein müssen, bei der Handhabung besteht hier die Gefahr, daß sich diese Zuleitungskabel verdrehen oder verknoten, außerdem ist die Handhabung schwierig, da bei beiden Branchen am Griffende Kabelanschlüsse vorgesehen werden müssen.

Es ist Aufgabe der Erfindung, ein gattungsgemäßes chirurgisches Instrument derart auszubilden, daß der Anschluß des Instrumentes an eine elektrische Versorgungsleitung vereinfacht werden kann.

Diese Aufgabe wird durch die Merkmale des Anspruchs 1 gelöst.

Auf diese Weise ist es möglich, beide elektrische Zuleitungen über ein und dieselbe Branche zu führen, eine Zuleitung wird durch die Branche selbst gebildet, eine weitere Zuleitung durch eine längs der Branche geführte, elektrisch von dieser isolierte Zuleitung. Dies ermöglicht es, die Versorgungsleitung des chirurgischen Instrumentes mit nur einer Branche zu verbinden, gegebenenfalls durch einen einzigen, zweipoligen Anschluß, die Versorgungsleitung kann also zweipolig bis zu dem Anschluß geführt werden, eine Verdrillungsgefahr oder eine Verknotungsgefahr entfallen, die andere Branche ist völlig frei von einem elektrischen Anschluß.

Es kann vorgesehen sein, daß die eine elektrische Zuleitung bildende Branche einstückig mit einem der Werkzeuge ausgebildet ist, dasselbe kann hinsichtlich der anderen Branche vorgesehen werden.

Die Werkzeuge können dabei Klemmbacken, Schneiden einer Schere oder ähnliches sein.

Die Lagerwelle kann die eine elektrische Zuleitung bildende Branche durchsetzen und ist in diesem Bereich von einer Isolierhülse umgeben, so daß die Lagerwelle gegenüber dieser Branche elektrisch isoliert ist.

Die Isolierhülse kann bei einer bevorzugten Ausführungsform einstückig mit einem zwischen den beiden Werkzeugen angeordneten, diese elektrisch voneinander isolierenden Isolierkörper ausgebildet sein.

Dabei ist es günstig, wenn die Isolierhülse und gegebenenfalls der Isolierkörper aus Keramik bestehen.

Vorzugsweise ist die Lagerwelle eine Schraube.

Bei einer ersten bevorzugten Ausführungsform ist vorgesehen, daß die eine elektrische Zuleitung bildende Branche rohrförmig ausgebildet ist und in ihrem Inneren elektrisch isoliert die weitere elektrische Zuleitung aufnimmt. Diese weitere elektrische Zuleitung kann als elektrische Leitung oder als dünner Stab ausgebildet sein, vorzugsweise ist diese weitere elektrische Zuleitung von einer Isolierhülle unmittelbar umgeben, so daß sie im Inneren der rohrförmigen Branche gegenüber dieser isoliert aufgenommen werden kann.

Die weitere elektrische Zuleitung kann an ihrem werkzeugseitigen Ende eine Öse tragen, durch die die Lagerwelle hindurchgesteckt ist. Auf diese Weise kann sehr einfach eine elektrische Verbindung zwischen Lagerwelle und der elektrischen Zuleitung hergestellt werden.

Günstig ist es weiterhin, wenn beide Branchen und Werkzeuge außenseitig von einer Isolierschicht umgeben sind, beispielsweise einer Isolierschicht aus einem sterilisierbaren Kunststoff wie Polyetheretherketon oder LCP (Flüssigkristallpolymer).

Die nachfolgende Beschreibung einer bevorzugten Ausführungsform der Erfindung dient im Zusammenhang mit der Zeichnung der näheren Erläuterung. Es zeigen:

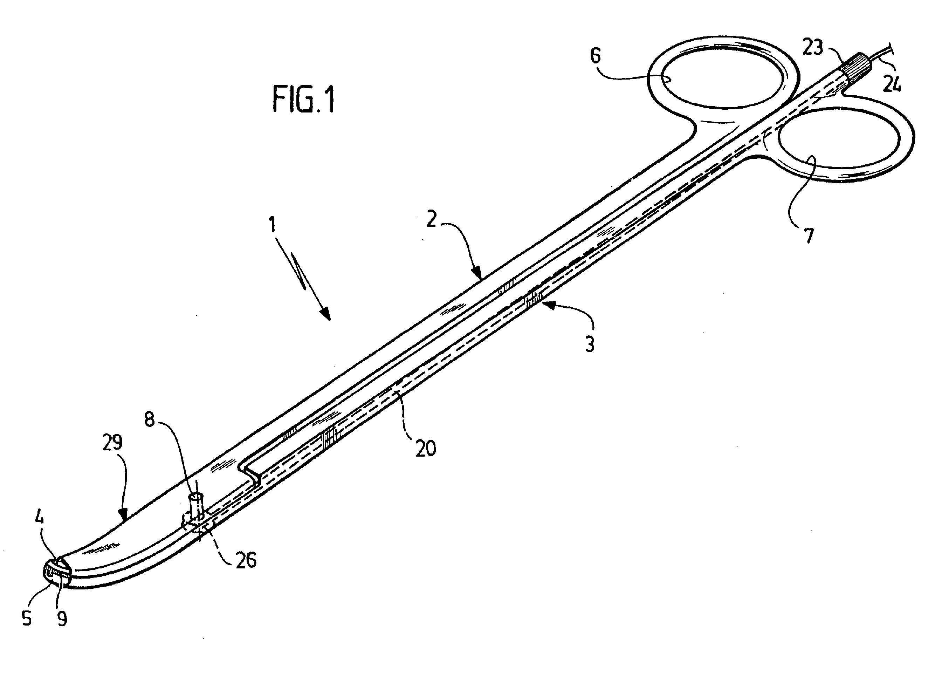

- Figur 1:: eine perspektivische Ansicht eines scherenförmigen chirurgischen Instrumentes mit einer zweipoligen elektrischen Zuleitung über eine der beiden Branchen und

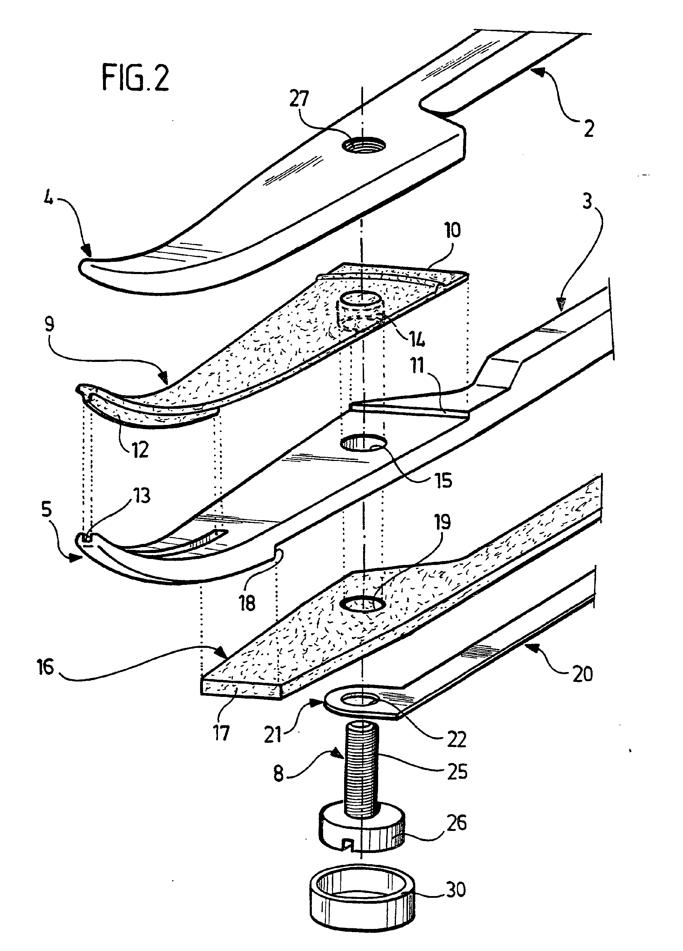

- Figur 2:: eine vergrößerte auseinandergezogene Teildarstellung des Instrumentes der Figur 1 im Bereich der Werkzeuge und der Schwenklagerung.

Das in der Zeichnung dargestellte chirurgische Instrument ist eine bipolare Schere 1 mit zwei länglichen Branchen 2, 3, die jeweils an ihrem distalen Ende in Form eines Schneidenblattes 4 bzw. 5 enden und die an ihrem proximalen Ende jeweils eine Grifföffnung 6 bzw. 7 aufweisen. Die beiden Branchen 2, 3 bestehen aus einem elektrisch leitenden Material, beispielsweise aus Stahl, und sind mittels einer sie quer durchsetzenden Lagerwelle 8 schwenkbar miteinander verbunden.

Auf eines der beiden Schneidenblätter 5 ist auf dessen dem anderen Schneidenblatt 4 zugekehrter Innenfläche ein dieses Schneidenblatt 5 vollständig bedeckender Isolierkörper 9 aus Keramik aufgelegt, dieser Isolierkörper 9 ist im wesentlichen als dünnes Plättchen ausgebildet, legt sich mit einer Endkante 10 an eine Stufe 11 des Schneidenblattes 5 an und greift mit einer Längsrippe 12 in eine korrespondierende Längsnut 13 des Schneidenblattes 5 ein. Einstückig mit dem Isolierkörper 9 ist eine Isolierhülse 14 verbunden, die von dem plättchenförmigen Isolierkörper 9 senkrecht absteht und in eine Lageröffnung 15 in der Branche 3 eintaucht, so daß diese Lageröffnung 15 an ihrer Innenseite vollständig von der Isolierhülse 14 abgedeckt wird. Der Isolierkörper 9 ist mit dem Schneidenblatt 5 dauerhaft verbunden, beispielsweise durch eine Verklebung oder eine Verlötung.

Auf der dem Isolierkörper 9 gegenüberliegenden Seite ist auf die Branche 3 ein weiterer, plattenförmiger Isolierkörper 16 aufgelegt, der mit einer Querkante 17 an einer Stufe 18 der Branche 3 anliegt und sich vom Bereich der Schwenklagerung der beiden Branchen 2, 3 bis an das den Grifföffnungen 6, 7 benachbarte Ende der Branche 3 erstreckt. Auch dieser im wesentlichen streifenförmige Isolierkörper 16 kann aus Keramik bestehen, im Bereich der Lageröffnung 15 weist er eine Durchstecköffnung 19 für die Lagerwelle 8 auf. Der Isolierkörper 16 ist mit der Branche 3 flächig verbunden, beispielsweise durch Verkleben oder Verlöten.

Außenseitig ist auf den Isolierkörper 16 eine elektrische Zuleitung in Form eines Metallstreifens 20 aufgelegt, der an seinem den Schneidenblättern 4, 5 benachbarten Ende einen quer abstehenden Vorsprung 21 mit einer Durchstecköffnung 22 aufweist, der weiterhin mit dem plattenförmigen Isolierkörper 16 flächig durch Verkleben oder Verlöten verbunden ist und der sich ebenfalls bis zum grifföffnungsseitigen Ende der Branche 3 erstreckt. An diesem Ende ist ein elektrischer Anschluß 23 vorgesehen, der ein zweipoliges, elektrisches Zuleitungskabel 24 sowohl mit der Branche 3 als auch mit dem Metallstreifen 20 derart verbindet, daß jeweils ein Pol des Zuleitungskabels 24 mit der Branche 3 und der andere Pol mit dem Metallstreifen 20 verbunden ist.

Die Lagerwelle 8 hat die Form einer Schraube mit einem Gewindeschaft 25 und einem Kopf 26, diese tritt durch die Durchstecköffnungen 22 und 19 hindurch, wird von der Isolierhülse 14 umgeben und ist in eine Lageröffnung 27 in der Branche 2 eingeschraubt. Die Lagerwelle 8 besteht aus Metall und verbindet auf diese Weise den Metallstreifen 20 elektrisch leitend mit der Branche 2, wobei sie die Branche 3 aufgrund der Isolierkörper 9 und 16 sowie der Isolierhülse 14 elektrisch isoliert durchsetzt.

Die beiden elektrischen Zuleitungen für die beiden Schneidenblätter 4 und 5 befinden sich somit ausschließlich an der Branche 3, diese bildet selbst eine der beiden elektrischen Zuleitungen, die andere wird durch den Metallstreifen 20 gebildet. Es ist daher auch möglich, das Zuleitungskabel 24 nur an dieser einen Branche 3 anzuschließen, so daß sich dadurch ein besonders gut handhabbares Instrument ergibt, bei dem es nicht notwendig ist, für jede Branche ein eigenes Anschlußkabel vorzusehen.

Beide Branchen 2 und 3 sind außenseitig mit einer Isolierbeschichtung 29 umgeben, die vorzugsweise aus einem sterilisierbaren Kunststoff besteht und die auf diese Weise alle Spannung führenden Teile nach außen hin abschirmt. Lediglich im distalen Endbereich der Schneidenblätter 4, 5 fehlt diese Isolierbeschichtung 29 und gibt dort die metallischen Schneidenblätter 4, 5 frei, die bei Anlage am Gewebe somit die an das Instrument angelegte Hochfrequenzspannung auf das zwischen den Schneidenblättern 4, 5 befindliche Gewebe übertragen können.

Die Abschirmung wird auch dadurch vervollständigt, daß der Kopf 26 der schraubenförmigen Lagerwelle 8 von einer Isolierkappe 30 überfangen wird.

## Patentansprüche

1. Scheren- oder zangenförmiges chirurgisches Instrument (1) mit zwei mittels einer Lagerwelle(8) schwenkbar miteinander verbundenen Branchen (2, 3), die jeweils ein zumindest teilweise aus einem elektrisch leitenden Material bestehendes Werkzeug (4, 5) tragen, wobei die Werkzeuge (4, 5) elektrisch voneinander isoliert sind und über elektrische Zuleitungen jeweils mit einem elektrischen Anschluß (23) an dem den Werkzeugen (4, 5) abgewandten Ende der Branchen (2, 3) in Verbindung stehen, wobei eine elektrische Zuleitung durch eine der beiden Branchen (3) gebildet wird und an derselben Branche (3) die weitere Zuleitung (20) von dieser Branche (3) elektrisch isoliert angeordnet ist und über die von dieser Branche (3) elektrisch isolierte, elektrisch leitende Lagerwelle (8) mit der anderen Branche (2) in Verbindung steht, **dadurch gekennzeichnet, daß** die eine elektrische Zuleitung bildende Branche (3) einseitig eine aus Keramik bestehende Isolierschicht (16) in Form einer dünnen Platte trägt, die sich zumindest von der Schwenklagerung bis zu den elektrischen Anschlüssen (23) erstreckt, und daß auf die Isolierschicht (16) die weitere elektrische Zuleitung in Form eines bandförmigen Leiterstreifens (20) aufgelegt ist.

2. Instrument nach Anspruch 1, **dadurch gekennzeichnet, daß** die eine elektrische Zuleitung bildende Branche (3) einstückig mit einem der Werkzeuge (5) ausgebildet ist.

3. Instrument nach einem der Ansprüche 1 oder 2, **dadurch gekennzeichnet, daß** die andere Branche (2) einstückig mit einem der Werkzeuge (4) ausgebildet ist.

4. Instrument nach einem der voranstehenden Ansprüche, **dadurch gekennzeichnet, daß** die Lagerwelle (8) die eine elektrische Zuleitung bildende Branche (3) durchsetzt und in diesem Bereich von einer Isolierhülse (14) umgeben ist.

5. Instrument nach Anspruch 4, **dadurch gekennzeichnet, daß** die Isolierhülse (14) einstückig mit einem zwischen den beiden Werkzeugen (4, 5) angeordneten, diese elektrisch voneinander isolierenden Isolierkörper (9) ausgebildet ist.

6. Instrument nach Anspruch 4 oder 5, **dadurch gekennzeichnet, daß** die Isolierhülse (14) und gegebenenfalls der Isolierkörper (9) aus Keramik bestehen.

7. Instrument nach einem der voranstehenden Ansprüche, **dadurch gekennzeichnet, daß** die Lagerwelle (8) eine Schraube ist.

8. Instrument nach einem der voranstehenden Ansprüche, **dadurch gekennzeichnet, daß** die weitere elektrische Zuleitung (20) an ihrem werkzeugseitigen Ende eine Öse (22) trägt, durch die die Lagerwelle (8) hindurchgesteckt ist.

9. Instrument nach einem der voranstehenden Ansprüche, **dadurch gekennzeichnet, daß** beide Branchen (2, 3) und Werkzeuge (4, 5) außenseitig von einer Isolierschicht (29) umgeben sind.

## Claims

1. A scissors- or forceps-shaped surgical instrument (1) with two branches (2, 3) pivotally connected to one another by means of a bearing shaft (8), each carrying a tool (4, 5) consisting at least partially of an electrically conductive material, the tools (4, 5) being electrically insulated from one another and being connected via electrical supply lines in each case to an electrical connection (23) at the end of the branches (2, 3) remote from the tools (4, 5), an electrical supply line being formed by one of the two branches (3) and the further supply line (20) being disposed on the same branch (3), electrically insulated from this branch (3), and via the electrically conductive bearing shaft (8) which is electrically insulated from this branch (3) is connected to the other branch (2), **characterised in that** the branch (3) forming an electrical supply line carries on one side an insulating layer (16) consisting of ceramics in the form of a thin plate which extends at least from the pivot mounting to the electrical connections (23), and **in that** the further electrical supply line in the form of a band-shaped conductor strip (20) is mounted on the insulating layer (16).

2. An instrument according to Claim 1, **characterised in that** the branch (3) forming an electrical supply line is designed as one piece with one of the tools (5).

3. An instrument according to one of Claims 1 or 2, **characterised in that** the other branch (2) is designed as one piece with one of the tools (4).

4. An instrument according to one of the preceding claims, **characterised in that** the bearing shaft (8) passes through the branch (3) forming an electrical supply line and in this region is enclosed by an insulating sleeve (14).

5. An instrument according to Claim 4, **characterised in that** the insulating sleeve (14) is designed as one piece with an insulating body (9) disposed between the two tools (4, 5) and electrically insulating them from one another.

6. An instrument according to Claim 4 or 5, **characterised in that** the insulating sleeve (14) and optionally the insulating body (9) consist of ceramics.

7. An instrument according to one of the preceding claims, **characterised in that** the bearing shaft (8) is a screw.

8. An instrument according to one of the preceding claims, **characterised in that** the further electrical supply line (20) carries at its tool-side end an eye (22) through which the bearing shaft (8) is inserted.

9. An instrument according to one of the preceding claims, **characterised in that** both branches (2, 3) and tools (4, 5) are enclosed externally by an insulating layer (29).

## Revendications

1. Instrument chirurgical (1) en forme de ciseaux ou de pince, comportant deux branches (2, 3) qui sont reliées l'une à l'autre de manière pivotante au moyen d'un arbre-palier (8) et portent chacune un outil (4, 5) constitué au moins partiellement par un matériau électroconducteur, dans lequel les outils (4, 5) sont électriquement isolés l'un par rapport à l'autre et se trouvent chacun en liaison via des lignes électriques d'amenée avec un branchement électrique (23) sur l'extrémité des branches (2, 3) détournée des outils (4, 5), dans lequel une ligne électrique d'amenée est formée à travers une des deux branches (3) et l'autre ligne d'amenée (20) est agencée sur la même branche (3) en étant isolée électriquement par rapport à cette branche (3) et reliée à l'autre branche (2) via l'arbre-palier (8) électroconducteur électriquement isolé par rapport à cette branche (3), **caractérisé en ce que** la branche (3) formant la ligne électrique d'amenée porte sur un côté une couche isolante (16) en céramique qui a la forme d'une plaque mince qui s'étend au moins depuis le montage pivotant jusqu'aux branchements électriques (23), et **en ce que** sur la couche isolante (16) est appliquée l'autre ligne électrique sous la forme d'une bande conductrice (20) sous forme de ruban.

2. Instrument selon la revendication 1, **caractérisé en ce que** la branche (3) formant la ligne électrique d'amenée est réalisée d'un seul tenant avec un des outils (5).

3. Instrument selon la revendication 1 ou 2, **caractérisé en ce que** l'autre branche (2) est réalisée d'un seul tenant avec un des outils (4).

4. Instrument selon l'une des revendications précédentes, **caractérisé en ce que** l'arbre-palier (8) traverse la branche (3) formant la ligne électrique d'amenée et est entourée dans cette région par une douille isolante (14).

5. Instrument selon la revendication 4, **caractérisé en ce que** la douille isolante (14) est réalisée d'un seul tenant avec un corps isolant (9) agencé entre les deux outils (4, 5) et isolant ceux-ci électriquement l'un de l'autre.

6. Instrument selon la revendication 4 ou 5, **caractérisé en ce que** la douille isolante (14) et, le cas échéant, le corps isolant (9) sont en céramique.

7. Instrument selon l'une des revendications précédentes, **caractérisé en ce que** l'arbre-palier (8) est une vis.

8. Instrument selon l'une des revendications précédentes, **caractérisé en ce que** l'autre ligne électrique d'amenée (20) porte à son extrémité côté outil un oeillet (22) à travers lequel passe l'arbre-palier (8).

9. Instrument selon l'une des revendications précédentes, **caractérisé en ce que** les deux branches (2, 3) et les outils (4, 5) sont entourés sur la face extérieure par une couche isolante (29).
